# FASCICULE DE BREVET EUROPEEN

(11) **EP 2 651 476 B1**
(45) Date de publication et mention de la délivrance du brevet: **28.10.2015**
(21) Numéro de dépôt: 11811080.8
(22) Date de dépôt: 12.12.2011
(51) Int. Cl.: A61M 15/00, B65D 25/54, B65D 81/30

(54) **INHALATEUR DE POUDRE SÈCHE**
TROCKENPULVERINHALATOR
DRY-POWDER INHALER

(30) Priorité: 14.12.2010 FR 1060451
(43) Date de publication de la demande: 23.10.2013
(73) Titulaire: Aptar France SAS, 27110 Le Neubourg (FR)
(72) Inventeur: BROUET, Guillaume, 76000 Rouen (FR); SALLAK, Zakaria, 76100 Rouen (FR)
(74) Mandataire: CAPRI
(86) Numéro de dépôt international: PCT/FR2011/052937
(87) Numéro de publication internationale: WO 2012/080635

(56) Documents cités:
- EP-A1- 0 825 223
- WO-A1-2005/089842
- WO-A1-2010/062744
- US-A1- 2005 016 543

## Description

La présente invention concerne un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. Un autre problème qui se pose avec les inhalateurs pourvus de bande de blisters est lié au déplacement de la bande, et au stockage de la partie utilisée de la bande. Ainsi, selon la longueur de la bande et/ou l'épaisseur des blisters, un espace important peut s'avérer nécessaire et tout blocage de la bande de blisters peut empêcher le bon fonctionnement de l'inhalateur. Par ailleurs, lorsque le dispositif d'avancée de la bande tire en même temps sur l'extrémité avant de la bande pour éviter un mauvais enroulement, il peut se poser un problème au fur et à mesure des actionnements en raison notamment du diamètre de la bande usée enroulée qui augmente progressivement. Les inhalateurs multidoses et les inhalateur contenant une bande de blisters sont donc généralement des dispositifs complexes, constituées d'un grand nombre de pièces, et donc coûteux à fabriquer et à assembler. Pour réaliser des dispositifs moins complexes et donc moins coûteux, il a été proposé des inhalateurs comportant des réservoirs individuels, tels que des capsules, qui sont à charger dans l'inhalateur juste avant l'utilisation de celui-ci. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. De plus, d'autres inconvénients spécifiques à ces inhalateurs à capsules sont apparus. Ainsi, ces dispositifs sont généralement constitués de deux pièces, l'une étant pourvue de l'embout buccal. Lors de la manipulation de ces dispositifs, pour ouvrir la capsule et libérer la poudre, ou pour vider la capsule vide après inhalation, les doigts de l'utilisateur entrent généralement en contact avec l'embout buccal, ce qui peut présenter des risques de contamination. De même, pour évacuer la capsule vide, le dispositif doit généralement être démonté, ce qui expose l'intérieur du dispositif à toute pollution extérieure, susceptible d'être par la suite transmise à l'utilisateur lors d'une future inhalation. De plus, les corps de ces inhalateurs à capsules sont généralement transparents pour permettre à l'utilisateur de voir l'intérieur de la chambre de dispersion, et ainsi savoir d'une part que la dose de poudre a bien été distribuée après utilisation, et d'autre part de vérifier si la capsule vide a été évacuée. Or, un tel corps transparent engendre certains inconvénients spécifiques. Ainsi, après chaque actionnement, un petit peu de poudre reste généralement collé aux parois de la chambre de dispersion. Ces résidus, visibles de l'extérieur, notamment après plusieurs utilisations, peuvent gêner l'utilisateur en donnant un aspect relativement sale au dispositif, ce qui peut parfois inciter l'utilisateur à ne plus vouloir utiliser l'inhalateur. De plus, de par sa transparence, un tel corps transparent laisse passer toute la lumière, et notamment les rayons ultraviolets qui peuvent altérer la poudre contenue dans la chambre de dispersion. Le document WO2010/062744 décrit un récipient contenant des composés photosensibles, le récipient comportant un corps interne en contact avec les composés photosensibles et un corps externe pourvu d'une fenêtre en matériau anti-UV.

La présente invention a pour but de fournir un inhalateur de poudre sèche, qui ne reproduit pas les inconvénients susmentionnés.

La présente invention a notamment pour but de fournir un tel inhalateur qui présente un aspect extérieur propre même après plusieurs utilisations.

En particulier, la présente invention a pour but de fournir un tel inhalateur qui soit simple et peu coûteux à fabriquer et à assembler, fiable d'utilisation, et limitant autant que possible les risques de contamination et/ou de pollution et/ou d'altération de la poudre avant inhalation.

La présente invention a donc pour objet un inhalateur de poudre sèche, comportant un corps formant une chambre de dispersion, un orifice de distribution à travers lequel l'utilisateur inhale, une ouverture de chargement recevant une capsule contenant une dose de poudre sèche à inhaler, et des moyens d'ouverture de capsule pour ouvrir une capsule insérée dans ladite ouverture de chargement et vider ladite dose de poudre dans ladite chambre de dispersion, ledit corps, au moins au niveau d'une paroi de ladite chambre de dispersion, étant réalisé en un matériau transparent teinté adapté à au moins partiellement filtrer les rayons ultraviolets et à permettre de voir l'intérieur de la chambre de dispersion tout en masquant au moins partiellement des résidus de poudre collés à ladite au moins une paroi en matériau transparent teinté.

Avantageusement, ledit matériau teinté comporte un coefficient d'absorbance élevé dans la plage des longueurs d'onde ultraviolettes, typiquement entre 10 et 400 nm.

Avantageusement, au moins un pigment est ajouté audit matériau pour le teinter.

Avantageusement, ledit au moins un pigment comporte des groupements chimiques chromophores.

Avantageusement, lesdits groupements chimiques chromophores absorbent les rayons ultraviolets lorsque mélangés audit matériau.

Avantageusement, lesdits groupements chimiques chromophores comprennent un ou plusieurs des éléments suivants: Ethene, 1-Hexyne, Ethanal, Nitromethane, bromure de méthyle, iodure de méthyle.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 est une vue schématique éclatée en perspective d'un dispositif de distribution selon un premier mode de réalisation avantageux,
- la figure 2 est une vue schématique en section transversale du dispositif de la figure 1, en position fermée avant la première utilisation,
- la figure 3 est une vue similaire à celle de la figure 2, en position ouverte, avec une capsule chargée dans l'ouverture de chargement,
- les figures 4 et 5 sont des vues similaires à celle de la figure 2, en cours de fermeture du capot et d'ouverture de capsule,
- la figure 6 est une vue similaire à celle de la figure 2, en position fermée, avant inhalation,
- la figure 7 est une vue similaire à celle de la figure 6, en position fermée, en cours d'inhalation,
- la figure 8 est une vue similaire à celle de la figure 3, en position ouverte,
- les figures 9 et 10 sont des vues schématiques en perspective du dispositif de la figure 1, respectivement en position fermée et ouverte,
- les figures 11 et 12 sont des vues schématiques en perspective découpée, respectivement en position fermée et ouverte,
- la figure 13 est une vue schématique éclatée en perspective d'un dispositif de distribution selon un second mode de réalisation avantageux,
- la figure 14a est une vue schématique en section transversale du dispositif de la figure 13, avant chargement de la capsule,
- la figure 14b est une vue schématique d'une surface d'extrémité axiale du corps, illustrant l'ouverture de vidage formé dans la position de la figure 14a,
- les figures 15a et 15b sont des vues similaires à celles des figures 14a et 14b, au début de l'ouverture de la capsule,
- les figures 16a et 16b sont des vues similaires à celles des figures 14a et 14b, encours d'ouverture de la capsule,
- la figure 17 est une vue similaire à celle de la figure 14a, après ouverture de la capsule et avant inhalation,
- la figure 18 est une vue similaire à celle de la figure 17, en cours d'inhalation,
- la figure 19 est une vue similaire à celle de la figure 18, selon une autre ligne de coupe,
- la figure 20 est une vue similaire à celle de la figure 18, après inhalation, et avant chargement de la prochaine capsule,
- la figure 21 est une vue similaire à celle de la figure 20, après chargement de la prochaine capsule, et
- la figure 22 est une vue schématique selon une autre section transversale du dispositif de la figure 13, montrant l'évacuation de la capsule vide dans la position de la figure 21.

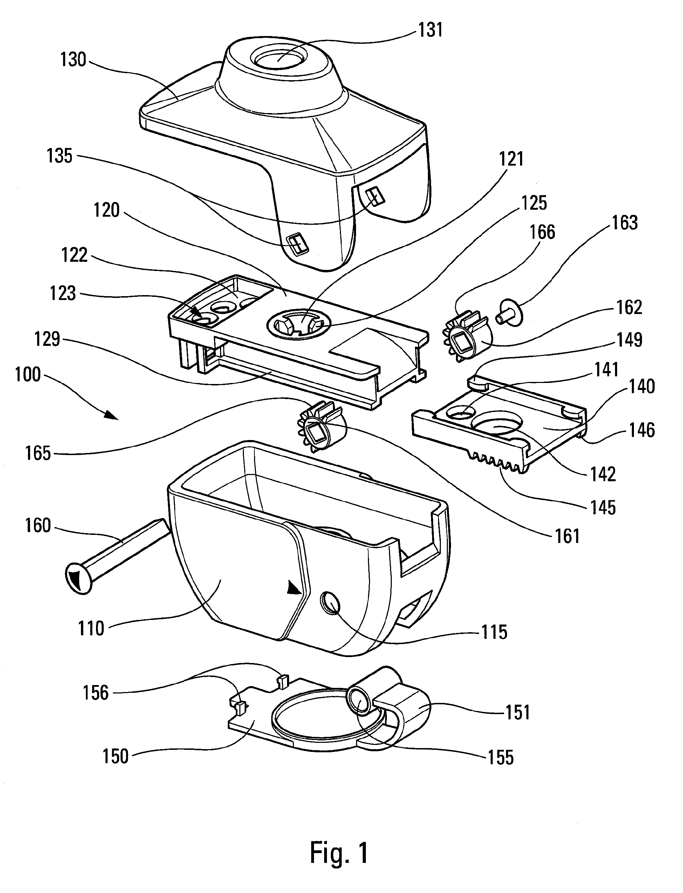

Les figures 1 à 12 décrivent un premier mode de réalisation d'un inhalateur. Dans ce premier mode de réalisation, l'inhalateur 100 comporte un corps 110 creux et comportant une ouverture supérieure et une ouverture inférieure. L'ouverture supérieure est au moins partiellement fermée par une partie de plaque 120 fixée sur ledit corps et l'ouverture inférieure est fermée par un élément de fermeture, tel qu'une trappe 150, monté pivotant sur ledit corps 110. Sous la partie de plaque 120 est interposé un organe coulissant 140 pourvu d'une denture. Avantageusement, la partie de plaque 120 comporte des moyens de guidage, tels que des rails 129, coopérant avec des projections 149 de l'organe coulissant 140, pour guider le déplacement en translation dudit organe coulissant. Un capot pivotant 130 est assemblé au-dessus de ladite partie de plaque 120. Ce capot 130 comporte l'orifice de distribution 131, formé de préférence au niveau d'un embout buccal autour duquel l'utilisateur placera sa bouche pour inhaler. Ainsi, comme visible notamment sur la figure 1, le dispositif selon ce premier mode de réalisation est constitué de cinq parties principales, à savoir le corps 110, la partie de plaque 120, l'organe coulissant 140, le capot 130 et la trappe 150. Tous ces éléments sont assemblés les uns sur les autres au moyen d'un axe 160 passant à travers une ouverture latérale 115 prévue dans le corps 110, à travers des ouvertures latérales appropriées 135 du capot 130 et à travers un cylindre creux 155 ménagé dans une partie de la trappe 150. Au moins un élément denté 161, 162 est monté sur ledit axe 160 pour coopérer avec au moins une denture 145 prévue sur l'organe coulissant 140. Dans l'exemple représenté, il y a deux éléments dentés 161 et 162 montés sur l'axe 160, et donc l'organe coulissant 140 comporte également deux dentures 145 et 146, dont le fonctionnement sera décrit ci-après. Un organe de fixation approprié 163 peut être prévu pour fixer de manière indémontable ledit axe 160 sur ledit corps 110 en assemblant les différentes parties constitutives les unes avec les autres. Comme visible sur la figure 1, l'axe 160 a de préférence une section particulière, par exemple sensiblement carrée, et les éléments dentés comportent également une section similaire de sorte qu'ils sont solidaires en rotation avec l'axe 160. De même le capot 130 comporte également des ouvertures 135 de forme similaire de sorte que le capot, les éléments dentés et ledit axe 160 sont solidaires en rotation. La partie de plaque 120 comporte une ouverture de chargement 121, avantageusement pourvue d'au moins une, de préférence trois nervures de positionnement 125, avantageusement réparties régulièrement autour de ladite ouverture de chargement 121. Ces nervures permettent de positionner et de maintenir de manière serrante une capsule 10 dans la position souhaitée. En particulier, les capsules 10 comportent une partie supérieure 11 et une partie inférieure 12 séparable de ladite partie supérieure, lesdites nervures 125 servent à maintenir ladite partie supérieure 11 avant et pendant la séparation de ladite partie inférieure 12. Par ailleurs, cette partie de plaque 120 comporte également avantageusement une zone de réservoir 122 formée par une zone pourvue d'une pluralité de trous 123 permettant de disposer une ou plusieurs capsule(s) de réserve. Ceci permet à l'utilisateur d'avoir toujours plusieurs capsules à disposition, par exemple lorsqu'il voyage. Dans cette hypothèse, après chaque utilisation du dispositif, il n'a plus qu'à se servir dans ce réservoir de capsule pour charger la prochaine capsule dans l'ouverture de chargement 121. Bien entendu, un tel réservoir de capsules n'est pas indispensable au fonctionnement du dispositif.

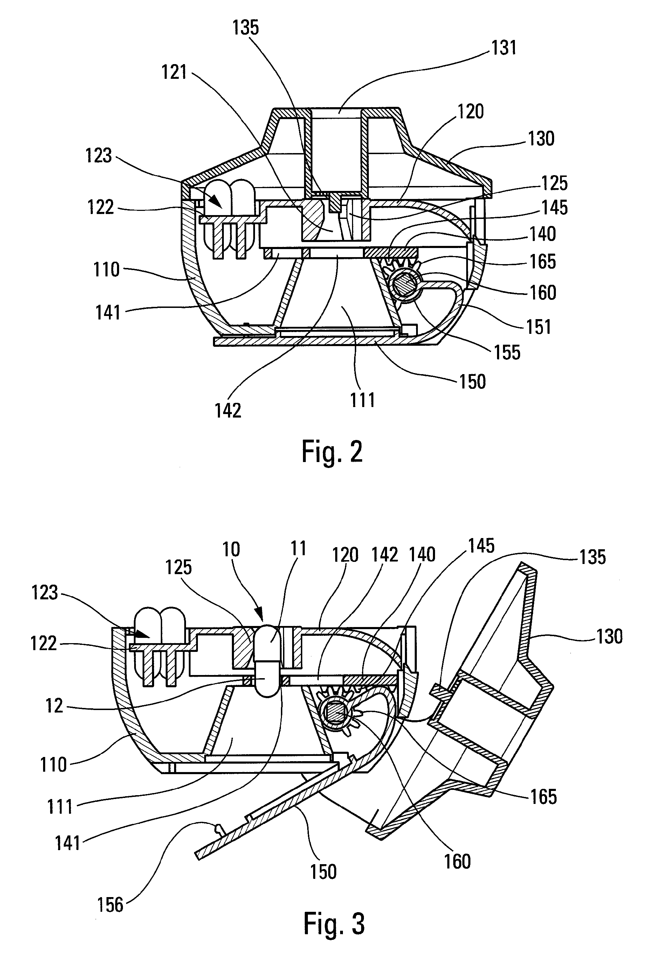

Les figures 2 à 8 illustrent un cycle de fonctionnement du dispositif selon ce premier mode de réalisation. En référence à la figure 2, qui montre le dispositif en position fermée avant la première utilisation, on constate que le corps 110 forme à l'intérieur une chambre de dispersion 111 qui sera destinée à recevoir la poudre après ouverture de la capsule 10. La figure 3 montre le dispositif après ouverture du capot 130. On constate que l'ouverture du capot 130 provoque la rotation de l'axe 160 due à la coopération de la forme environ carrée de cet axe 160 avec les orifices de forme correspondante 135 dudit capot 130. Cette rotation de l'axe 160 provoque donc également une rotation des éléments dentés 161 et 162 qui tournent avec ledit axe 160. Une rotation de ces éléments dentés 161, 162 provoque une translation latérale de l'organe coulissant 140. En effet, comme notamment représenté sur la figure 2, la denture 165 de l'élément denté 161 est engrenée dans la denture 145 de l'organe coulissant 140. Ainsi entre les figures 2 et 3, on constate que la rotation de la denture 165 de l'élément denté va faire coulisser l'organe coulissant 140 vers la droite sur les figures. Bien entendu, il se passe la même chose de l'autre côté du dispositif avec le second élément denté 162, non visible sur les figures en coupe. Bien entendu, un seul élément denté peut être suffisant pour provoquer le déplacement dudit organe coulissant. Lors de l'ouverture du capot 130, la trappe 150 ne tourne pas avec l'axe 160. Par contre, en fin d'ouverture du capot 130, celui-ci coopère avec ladite trappe 150, et notamment avec une partie courbe 151 reliant le cylindre creux 155, qui est monté sur l'axe 160, à la partie de la trappe qui obture le fond du corps 110 en position de fermeture de la trappe. Cette coopération entre le capot 130 et la trappe 150 fait basculer la trappe autour dudit axe 160 vers la position ouverte représentée sur la figure 3. Ainsi, dans cette position totalement ouverte du capot 130, la trappe 150 est ouverte et l'intérieur de la chambre de dispersion 111 peut être évacué hors du dispositif. La figure 3 montre également une capsule 10 mise en place à l'intérieur de l'ouverture de chargement 121. On constate que la partie supérieure 11 de la capsule est maintenue de manière serrante dans les nervures 125 prévues dans ladite ouverture de chargement 121. Par ailleurs, la partie inférieure 12 de la capsule passe à travers une première ouverture 141 réalisée dans ledit organe coulissant 140 et qui, dans la position ouverte du capot 130, se trouve en face de ladite ouverture de chargement 121.

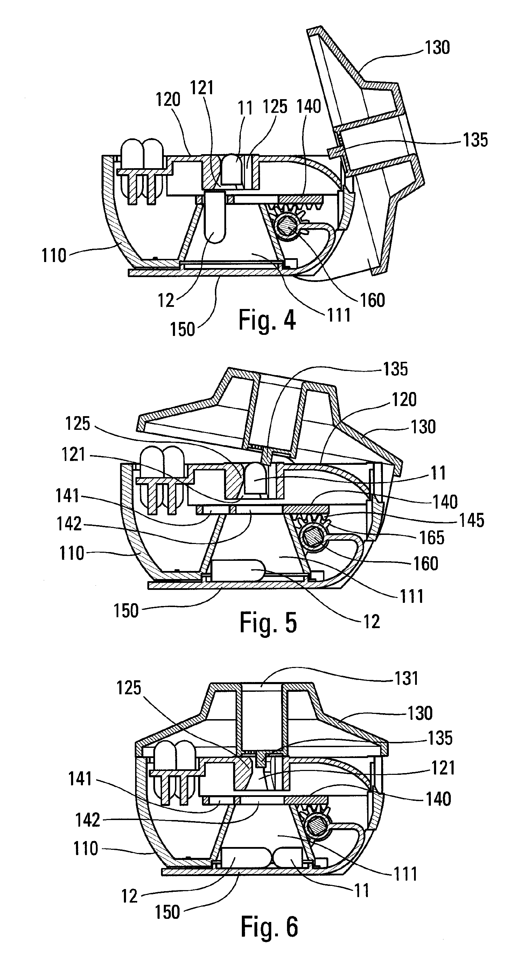

Les figures 4 à 6 illustrent la phase de fermeture du capot 130 après chargement de la capsule 10. Ainsi, comme visible sur la figure 4, lorsque l'utilisateur referme le capot 130, la trappe 150 va également se refermer et l'organe coulissant 140 va être ramené vers la gauche sur les figures par la coopération entre les éléments dentés 161 et 162 et les dentures dudit organe coulissant 140. Or, la partie inférieure 12 de la capsule 10 passant à travers la première ouverture 141 dudit organe coulissant 140, un déplacement latéral de cet organe coulissant va casser la partie inférieure 12 de la capsule 10 comme illustré sur la figure 4. L'organe coulissant 140 agit donc en tant que moyen d'ouverture de capsule. La partie supérieure 11 de la capsule 10 reste bien entendu maintenue de manière serrante dans l'ouverture de chargement 121, notamment par les nervures 125. Par contre, la partie inférieure 12 va tomber dans la chambre de dispersion 111, puisque le premier orifice 141 dudit organe coulissant 140 a un diamètre plus large que le diamètre externe de ladite partie inférieure 12 de la capsule. Ainsi, non seulement la poudre va se vider à l'intérieur de la chambre de dispersion 111, mais la partie inférieure 12 contenant ladite poudre va tomber sur le fond de ladite chambre de dispersion 111, pour permettre un vidage de celle-ci. Dans la position de figure 5, on constate que le capot 130 arrive juste avant sa position de fermeture. Dans cette position, un ergot 135 prévu dans ladite partie de capot 130 va coopérer avec la partie supérieure 11 de la capsule 10 qui est restée dans l'ouverture de chargement 121. Ainsi, et comme clairement illustré sur les figures 5 et 6, lors de la fermeture totale du capot 130, l'ergot 135 va provoquer l'expulsion de la partie supérieure 11 de la capsule depuis l'ouverture de chargement 121 vers l'intérieur de la chambre de dispersion 111. Dans cette position de la figure 6, où le dispositif est à nouveau complètement fermé, la capsule 10 a donc été cassée en deux, les deux parties supérieure 11 et inférieure 12 de la capsule 10 reposant dans la chambre de dispersion 111 sur le fond (formé par la trappe 150) et avec la poudre au moins partiellement expulsée hors desdites parties de capsules. Le dispositif est alors prêt pour l'inhalation.

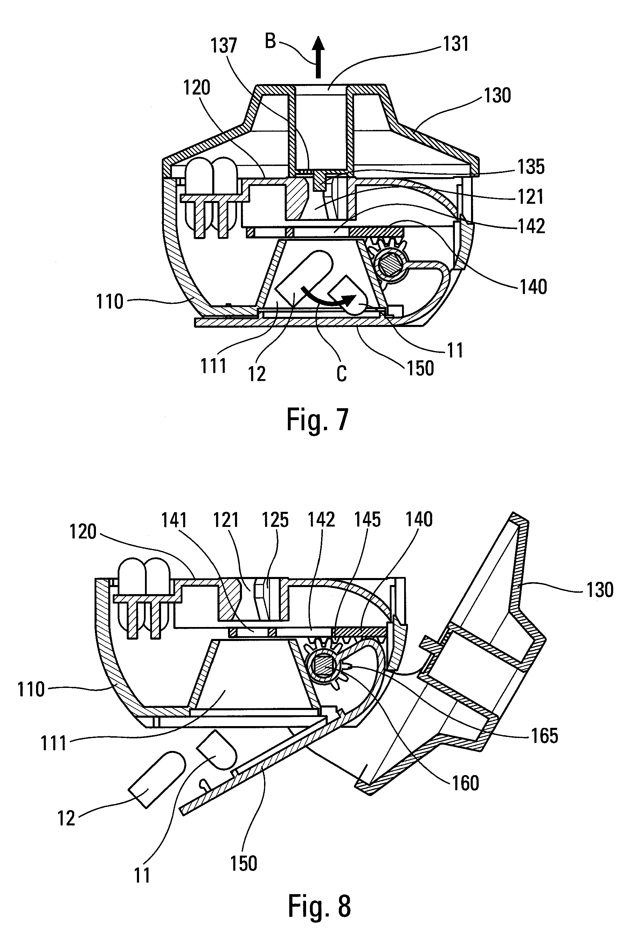

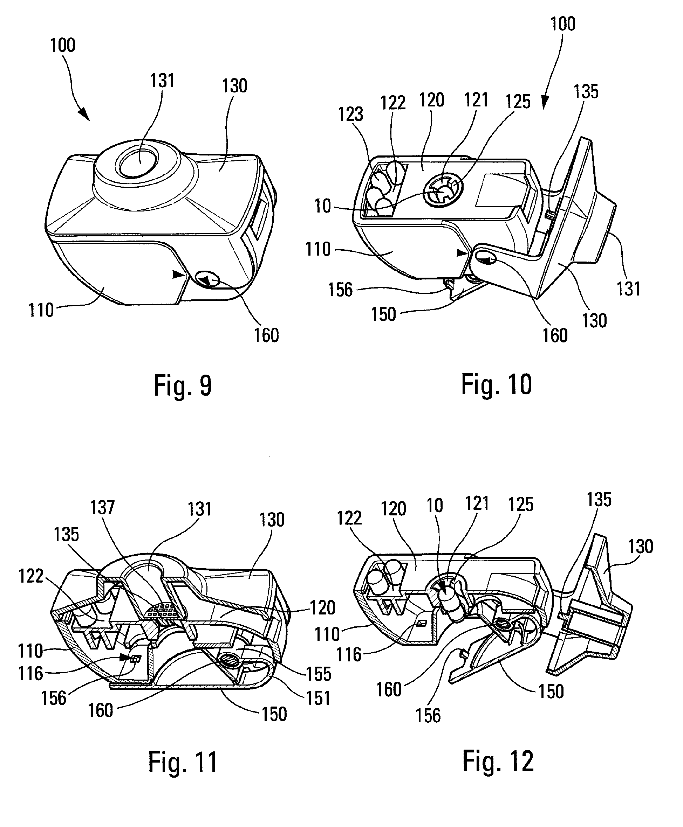

La figure 7 illustre la phase d'inhalation. Pour inhaler, l'utilisateur place sa bouche autour de l'orifice de distribution 131 du capot 130 et aspire dans le sens de la flèche B, représenté sur la figure 7. Ce faisant, il crée un courant d'air à l'intérieur de la chambre de dispersion 111 qui va faire tourbillonner les deux parties de capsules 11 et 12 à l'intérieur de ladite chambre de dispersion 111. Ce tourbillonnement illustré par la flèche C sur la figure 7 va permettre un vidage total desdites parties de capsules mais également une bonne dispersion de la poudre, et notamment une désagglomération des éventuels amas de poudre qui auraient pu se former. Eventuellement, des entrées d'air supplémentaires peuvent être prévues dans la chambre de dispersion pour favoriser le tourbillonnement du flux d'inhalation. La poudre qui tourbillonne va alors être expulsée hors de la chambre de dispersion 111 par le flux d'inhalation à travers une seconde ouverture 142 prévue dans l'organe coulissant 140 et qui, dans cette position d'inhalation, se trouve face d'une part à la chambre de dispersion 111 et d'autre part à l'ouverture de chargement 121. Comme visible plus clairement sur la figure 11, le capot 130 comporte avantageusement une grille 137 à travers laquelle la poudre va pouvoir passer et être expulsée en direction de l'orifice de distribution 131. La grille évite notamment que les parties de capsules 11, 12 ne soient également expulsées hors de la chambre de dispersion. L'utilisateur inhale alors la dose de poudre qui était initialement contenue dans la capsule 10. Avantageusement, ladite chambre de dispersion peut avoir une forme tronconique se rétrécissant en direction de l'orifice de distribution 131, pour notamment accélérer le flux d'inhalation en direction dudit orifice.

Après inhalation, l'utilisateur ouvre à nouveau le capot 130 ce qui, comme précédemment, va faire basculer la trappe 150 en fin d'ouverture. Ce basculement de la trappe 150 illustré sur la figure 8, va permettre d'évacuer les deux parties de capsules 11 et 12 vides de la chambre de dispersion 111. Dans cette position de la figure 8, le dispositif est donc prêt pour une prochaine utilisation. Bien entendu, si cette prochaine utilisation n'est pas immédiate, l'utilisateur peut refermer le dispositif et attendre de le rouvrir la prochaine fois qu'il en a besoin. En variante, l'utilisateur n'est pas obligé de vider les parties de capsules vides après chaque inhalation mais il peut bien entendu le faire seulement lors de la prochaine ouverture du dispositif lorsqu'il veut charger une nouvelle capsule.

Avantageusement, comme illustré notamment sur la figure 1, la trappe 150 peut comporter un ou plusieurs ergot(s) de fixation 156 qui s'encliquète(nt) légèrement dans le fond du corps 110 en position de fermeture, pour garantir une fermeture sûre et fiable de la trappe 150 en position fermée. Les figures 11 et 12 montrent des ouvertures 116 ménagées dans le fond du corps 110 à travers lesquelles lesdits ergots d'encliquetage 156 de la trappe vont pouvoir passer. Bien entendu, cet encliquetage n'est pas trop fort pour ne pas gêner l'ouverture de la trappe lorsque l'utilisateur ouvre le capot 130.

Les figures 9 et 10 montrent des vues en perspective du dispositif en positions fermée et ouverte, et les figures 11 et 12 sont des vues similaires aux figures 9 et 10 mais partiellement découpées, montrant la structure interne du dispositif dans ces deux positions.

Le dispositif est donc particulièrement simple et astucieux. Il est constitué d'un faible nombre de pièces, il est donc peu coûteux à fabriquer et à assembler. Par ailleurs, la présence d'une chambre de dispersion et des parties de capsules vides qui tourbillonnent permettent de désagglomérer la poudre et garantissent donc une meilleure distribution de celle-ci à l'utilisateur lors de l'inhalation. Enfin, l'évacuation des parties de capsules vides ne nécessite pas un démontage du dispositif ce qui limite les risques de pollution de celui-ci. L'absence de démontage du dispositif évite aussi les risques de ne plus pouvoir le remonter, ou d'égarer les parties démontées, notamment chez des enfants ou des personnes âgées. De plus, la manipulation du dispositif, c'est-à-dire l'ouverture et la fermeture du capot 130, n'impose pas de manipuler la partie formant l'embout buccal autour de l'orifice de distribution 131. Eventuellement, on pourrait envisager une partie de préhension spécifique pour la manipulation dudit capot. Les risques de contamination au niveau de l'orifice de distribution 131 sont donc également limités. Le procédé d'utilisation du dispositif est donc très simple, l'utilisateur n'ayant qu'à déplacer le capot entre ses deux positions d'extrémité pour complètement actionner le dispositif. Ainsi, il ouvre d'abord le capot, puis il insère une capsule, il referme le capot et il inhale.

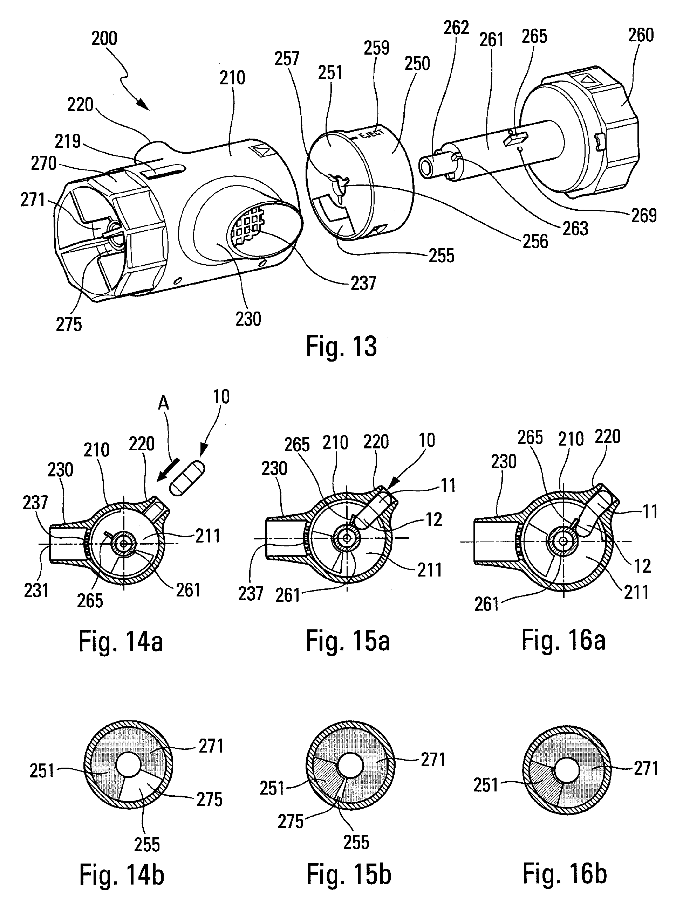

Les figures 13 à 22 illustrent un second mode de réalisation d'un inhalateur. La figure 13 illustre notamment une vue en perspective éclatée du dispositif. Dans ce second mode de réalisation, l'inhalateur 200 n'est constitué que de trois pièces. Un corps 210 de forme sensiblement cylindrique est pourvu sur sa périphérie d'un embout buccal 230 définissant l'orifice de distribution et d'une ouverture de chargement 220 adaptée à recevoir une capsule 10. Ce corps 210 peut comporter un axe central longitudinal. L'intérieur du cylindre 210 forme une chambre de dispersion 211. Une première partie d'extrémité axiale dudit corps 210 est formée par une première partie de préhension 270, fixe par rapport au corps 210. Cette première partie de préhension pourrait être simplement formée par un bord axial dudit corps. Avantageusement, comme représenté, cette première partie de préhension 270 présente un profil externe particulier, pour inciter l'utilisateur à manipuler le dispositif par son intermédiaire. Cette première partie de préhension 270 comporte une paroi de fermeture 271 de la chambre de dispersion 211, ladite paroi de fermeture 271 comportant une ouverture 275, s'étendant par exemple sur un angle d'environ 60° à 90° dans ladite paroi de fermeture 271. De l'autre coté, le corps 210 est ouvert, et cette ouverture est fermée par une seconde partie de préhension 260 montée rotative par rapport audit corps 210. Cette seconde partie de préhension 260 forme donc la seconde partie d'extrémité axiale du corps. Avantageusement, cette seconde partie de préhension 260 présente un profil externe similaire à celui de la première partie de préhension 270. L'utilisateur est donc naturellement incité à saisir chaque partie de préhension 260, 270 avec une main respective, et de faire tourner l'une par rapport à l'autre pour manipuler le dispositif. A l'intérieur du corps 210 cylindrique, du coté de cette première partie d'extrémité axiale, est disposé un élément de fermeture, tel qu'un organe de trappe 250, comportant également une paroi axiale 251 pourvue d'une fenêtre 255 dont les dimensions correspondent environ à la fenêtre 275 réalisée dans la paroi de fermeture 271 du corps 210. Cet organe de trappe 250 est monté solidaire en rotation sur un axe central 261 raccordé à ladite seconde partie de préhension 260. Par exemple, des nervures 263 formée sur l'extrémité 262 de l'axe central 261 coopèrent avec des rainures 257 dudit organe de trappe 250, comme illustré sur la figure 13. L'organe de trappe 250 est donc solidaire en rotation avec la seconde partie préhension 260 du dispositif. L'organe de trappe 250 est disposé à l'intérieur du corps 210 pour coopérer avec la paroi de fermeture 271 de celui-ci. Ainsi, pour utiliser le dispositif de ce second mode de réalisation l'utilisateur agrippe avec ses deux mains les deux parties de préhension 260 et 270 et fait tourner l'une par rapport à l'autre, comme cela sera expliqué plus en détail ci-après. A aucun moment, l'utilisateur n'est obligé de toucher la partie d'embout buccal 230 pour utiliser le dispositif.

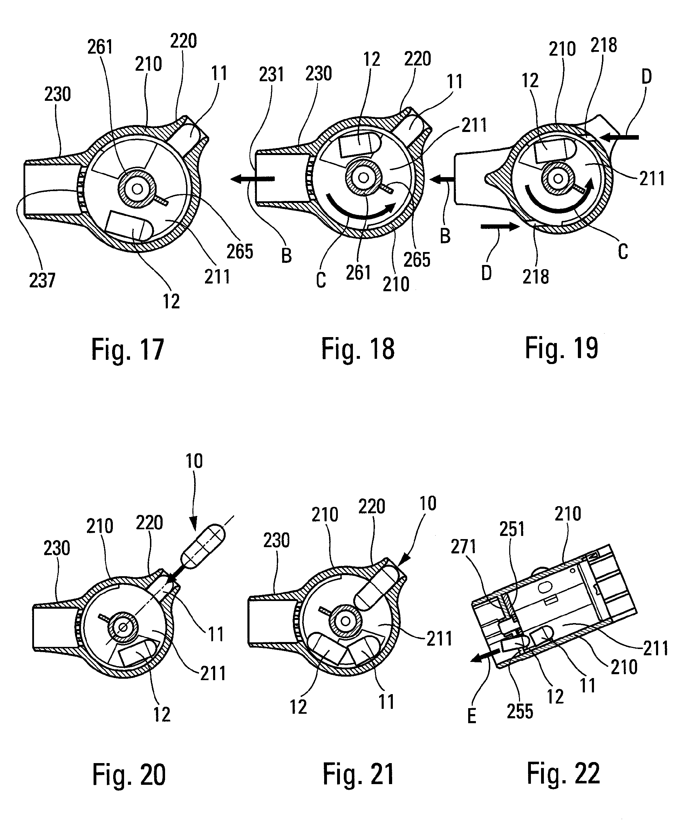

Les figures 14a, 15a et 16a illustrent une phase de chargement et d'ouverture de capsule 10. Ainsi, en se référant plus particulièrement à la figure 14a, on voit une section transversale à travers le corps 210 et donc on voit la chambre de dispersion 211, l'ouverture de chargement 220 et l'embout buccal 230, avec au centre l'axe central 261 qui est monté rotatif dans ladite chambre de dispersion 211. L'utilisateur charge une capsule 10 selon la flèche A dans l'ouverture de chargement 220. La profondeur dudit orifice 220 est ajustée pour que lorsque l'utilisateur insère entièrement la capsule 10 dans ladite ouverture de chargement 220, la partie supérieure 11 de la capsule est maintenue serrée dans ladite ouverture de chargement 220 alors que la partie inférieure 12 de la capsule se projette à l'intérieur de la chambre de dispersion 211. L'utilisateur va alors tourner la seconde partie de préhension 260 par rapport à la première partie de préhension 270, et donc par rapport au corps 210. Cette rotation est illustrée sur les figures 15a et 16a. Comme visible notamment sur la figure 13, l'axe central 261 est pourvu d'une projection 265, par exemple en forme de languette. Comme visible sur les figures 15a et 16a, lors de la rotation de la seconde partie de préhension 260 par rapport au corps 210, ladite projection 265 va venir en contact de la partie inférieure 12 de la capsule. La figure 15a montre la position juste avant que la capsule ne soit ouverte alors que la figure 16a montre la capsule en cours d'ouverture, avec la projection 265 qui pousse sur la partie inférieure 12 de la capsule. On voit que la rotation du premier axe 261 provoque la rotation de la projection 265, qui va déformer la partie inférieure de capsule, laquelle va donc se séparer de la partie supérieure 11 de la capsule, qui reste coincée dans l'ouverture de chargement 220. La figure 17 illustre la position de la capsule 10 ouverte avec la partie supérieure 11 coincée dans l'ouverture de chargement 220 et la partie inférieure 12 qui est tombée librement dans la chambre de dispersion 211 pour se vider dans celle-ci. La projection 265 agit donc en tant que moyen d'ouverture de capsule. Les figures 14b, 15b et 16b illustrent ce qui se passe au niveau de la première partie d'extrémité axiale du corps, et en particulier au niveau de la paroi de fermeture 271 de la chambre de dispersion. Ainsi, dans la position de la figure 14a, au moment du chargement de la capsule 10, la fenêtre 255 de la paroi axiale 251 de l'organe de trappe 250 est en face de la fenêtre 275 de la paroi de fermeture 271. Lorsque l'utilisateur va faire tourner la seconde partie de préhension 260 il va également faire tourner l'organe de trappe 250 puisque celui-ci est solidaire en rotation de l'élément de manipulation 260. La partie de paroi 251 va donc progressivement obturer l'ouverture 275 de la paroi de fermeture axiale 271 du corps 210. La figure 15b montre que juste avant que la capsule ne commence à être ouverte, il y a encore un petit passage ouvert mais que dès que la capsule est déformée, ce qui est représenté sur la figure 16b, l'ouverture est totalement fermée et l'intérieur de la chambre de dispersion 211 est donc fermé. Ainsi, au moment où la capsule se casse et la poudre se vide dans la chambre de dispersion 211, celle-ci est fermée au niveau de ses parois d'extrémités axiales.

L'utilisateur peut alors inhaler comme illustré par la flèche B sur la figure 18. Pour ce faire, il place sa bouche autour de l'embout buccal et va créer un flux d'inhalation qui va lui permettre d'inhaler la poudre contenue dans la chambre de dispersion 211 à travers l'orifice de distribution 231. La forme sensiblement annulaire de la chambre de dispersion 211 autour de l'axe central 261 est avantageuse en ce qu'elle favorise le tourbillonnement du flux d'inhalation provenant de l'embout buccal. Comme dans le premier mode de réalisation, l'embout buccal comporte de préférence une grille 237 pour laisser passer la poudre mais empêcher les parties de capsule d'être expulsées dans la bouche de l'utilisateur. Le flux d'inhalation créé par l'utilisateur va faire tourbillonner la partie de capsule inférieure 12 qui tourne librement à l'intérieur de la chambre de dispersion. A nouveau, ceci assure le vidage de ladite partie inférieure de capsule et permet une bonne dispersion et désagglomération de la poudre au moment de sa distribution à l'utilisateur. Ce tourbillonnement est illustré par la flèche C sur la figure 18. Comme visible sur la figure 19 qui montre le dispositif selon une ligne de coupe légèrement décalée, on constate que la chambre de dispersion 211 comporte une ou plusieurs ouverture(s) tangentielle(s) 218 ménagée(s) dans le corps 210. Ceci favorise le tourbillonnement du flux d'inhalation puisque l'utilisateur au moment où il inhale va aspirer des flux d'air à travers ces ouvertures tangentielles 218 (flèches D) qui vont donc naturellement tourner à l'intérieur de ladite chambre de dispersion 211, et ainsi faire tourbillonner encore davantage la partie inférieure 12 de la capsule pour disperser et désagglomérer la poudre.

Avantageusement, l'axe central 261 comporte des ouvertures 269, par exemple disposées autour de la projection 265. Ceci est illustré sur la figure 13, mais d'autres ouvertures 269 pourraient aussi être prévues le long de l'axe 261. Ces ouvertures ont un double effet. D'une part, elles permettent des entrées d'air supplémentaires lors de l'inhalation et créent donc encore des flux différents qui favorisent encore davantage le vidage de la capsule, le tourbillonnement et la désagglomération de la poudre. D'autre part, les trous disposés à proximité de la projection 265 garantissent que la partie inférieure 12 de la capsule ne vient pas s'emboîter sur ladite projection 265 en y piégeant de la poudre. Dès que l'utilisateur va inhaler, le flux d'air qui va passer à travers lesdits orifices 269 va expulser ladite partie de capsule dans l'hypothèse où elle serait emboîtée sur ladite projection 265.

Après l'inhalation, l'utilisateur ramène de préférence le dispositif dans sa position de départ, en faisant tourner la seconde partie de préhension 260 en sens inverse par rapport au corps. Ce faisant, il va ouvrir à nouveau la fenêtre 275 de la paroi de fermeture axiale 271, et ainsi permettre l'évacuation de la partie inférieure 12 de la capsule à travers les fenêtres alignées 255 et 275. Lorsque l'utilisateur vient charger la prochaine capsule 10 dans l'ouverture de chargement 220, comme illustré sur la figure 20, il va pousser la partie supérieure 11 de la précédente capsule, qui était restée coincée dans l'ouverture de chargement 220, à l'intérieur de la chambre de dispersion 211. Celle-ci pourra alors aussi être évacuée hors de la chambre de dispersion. Ceci est illustré sur les figures 21 et 22. En effet, dans cette position où les fenêtres 255 et 275 sont l'une face à l'autre, l'utilisateur peut simplement incliner le dispositif et évacuer la ou les parties de capsule à travers lesdites fenêtres. Avantageusement, pour inciter l'utilisateur à charger la prochaine capsule dans la première position, à savoir celle ou la chambre de dispersion est ouverte, le corps 210 comporte une fenêtre 219 et l'organe de trappe 250 comporte une indication 259, par exemple le mot EJECT, qui vient s'afficher dans ladite fenêtre 219 lorsque les deux fenêtres 255, 275 sont l'une en face de l'autre. Eventuellement, dans la seconde position d'extrémité, c'est-à-dire dans la position où la capsule a été ouverte, le mot INHALE ou similaire pourra être affiché dans la fenêtre 219 pour indiquer à l'utilisateur qu'il est dans la position dans laquelle il peut inhaler. Des repères visuels peuvent aussi être prévus sur la seconde partie de préhension 260 et/ou le corps 210, pour indiquer visuellement à l'utilisateur les deux positions d'extrémité de ladite seconde partie de préhension par rapport audit corps.

Ce second mode de réalisation permet donc de réaliser un dispositif où l'utilisateur n'a pas besoin de manipuler l'embout buccal pour utiliser le dispositif. De plus, il n'a pas non plus besoin de le démonter le dispositif pour expulser ou évacuer les parties de capsules vides après chaque utilisation. Les risques de contamination et de pollution sont donc fortement limités, de même que les risques de pertes de parties constitutives démontées ou encore ceux de ne plus pouvoir remonter le dispositif après démontage. Ce second mode de réalisation est encore plus simple que le premier, puisqu'il ne comporte que trois pièces. Il permet de garantir une bonne dispersion de la poudre, d'une part en la désagglomérant de manière appropriée grâce à la partie de capsule qui tourbillonne dans la chambre de dispersion, mais également grâce aux arrivées d'air supplémentaires ménagées dans le corps 210 et/ou l'axe central 261. Le procédé d'utilisation du dispositif est également très simple, l'utilisateur n'ayant qu'à déplacer la seconde partie de préhension entre ses deux positions d'extrémité pour complètement actionner le dispositif. Ainsi, il insère d'abord une capsule, puis il fait tourner la seconde partie de préhension vers sa seconde position d'extrémité, il inhale, et il ramène la seconde partie de préhension vers sa première position d'extrémité.

Selon l'invention, la partie du corps qui forme au moins une paroi de la chambre de dispersion est réalisée en un matériau transparent teinté. Ce matériau teinté est adapté d'une part à au moins partiellement filtrer les rayons ultraviolets, pour éviter toute dégradation ou altération de la poudre contenue dans ladite chambre. Ceci s'applique à la poudre contenue dans la chambre de dispersion avant chaque inhalation, mais aussi aux résidus de poudre qui restent accrochés ou collés à la paroi de la chambre de dispersion après chaque inhalation, et qui sont susceptibles d'être au moins partiellement inhalés lors des inhalations suivantes. D'autre part, l'utilisation d'un matériau transparent teinté permet une visibilité partielle de l'intérieur de la chambre de dispersion. Ceci permet à l'utilisateur de voir que la dose de poudre a été distribuée et/ou que la capsule vide a été évacuée, mais évite l'aspect un peu sale en masquant au moins partiellement lesdits résidus de poudre restants accrochés ou collés aux parois de la chambre de dispersion. Selon la présente invention, c'est donc la pièce qui contient directement la poudre qui est au moins en partie réalisée en matériau transparent teinté.

Le caractère teinté pourrait être caractérisé techniquement par le coefficient d'absorbance. Ce coefficient d'absorbance est une grandeur sans unité qui représente le rapport entre le flux incident et le flux transmis par le matériau. Ainsi, un matériau anti UV aura un bon coefficient d'absorbance dans la plage des longueurs d'ondes ultraviolettes, à savoir entre environ 10nm et 400nm. Des pigments peuvent être ajoutés au matériau pour favoriser ses caractéristiques, notamment anti UV. Ces pigments comportent des groupements chimiques appelés chromophores qui absorbent les rayons ultraviolets, par exemple un ou plusieurs des éléments suivants : Ethene, 1-Hexyne, Ethanal, Nitromethane, bromure de méthyle, iodure de méthyle. Les chromophores sont présents dans les pigments, c'est ce qui leur confère une couleur. Il s'agit souvent de double liaisons carbone-carbone ou oxygène, mais il en existe d'autres types. Ces groupements sont ensuite incorporés dans la matière. C'est leur proportion en pourcentage qui confère la couleur voulue mais donne également ses propriétés différentes au mélange.

Le matériau est avantageusement un matériau synthétique tel que par exemple du COC (Cyclo Oléfine Copolymère) ou COP (Cyclo Oléfine Polymère, du PP (Polypropylène), du SAN (Styrène Acrylonitrile) ou, de préférence, du PCTA (Polycyclohexylènediméthylène Téréphthalate). Des alliages de ces matériaux entre eux ou avec d'autres matériaux appropriés sont aussi possibles.

Diverses modifications sont également possibles pour un homme du métier sans sortir du cadre de la présente invention tel que défini par les revendications annexées. En particulier, les diverses caractéristiques et fonctionnalités du dispositif décrites en référence aux dessins peuvent être combinées entre elles d'une quelconque façon appropriée.

## Revendications

1. Inhalateur de poudre sèche (100 ; 200), comportant un corps (110 ; 210) formant une chambre de dispersion (111 ; 211), un orifice de distribution (131 ; 231) à travers lequel l'utilisateur inhale, une ouverture de chargement (121 ; 220) recevant une capsule (10) contenant une dose de poudre sèche à inhaler, et des moyens d'ouverture de capsule (140 ; 265) pour ouvrir une capsule insérée dans ladite ouverture de chargement et vider ladite dose de poudre dans ladite chambre de dispersion, **caractérisé en ce que** ledit corps (110 ; 210), au moins au niveau d'une paroi de ladite chambre de dispersion, est réalisé en un matériau transparent teinté adapté à au moins partiellement filtrer les rayons ultraviolets, et à permettre de voir l'intérieur de la chambre de dispersion tout en masquant au moins partiellement des résidus de poudre collés à ladite au moins une paroi en matériau transparent teinté.

2. Inhalateur selon la revendication 1, dans lequel ledit matériau teinté comporte un coefficient d'absorbance élevé dans la plage des longueurs d'onde ultraviolettes, typiquement entre 10 et 400 nm.

3. Inhalateur selon la revendication 1 ou 2, dans lequel au moins un pigment est ajouté audit matériau pour le teinter, ledit au moins un pigment comportant des groupements chimiques chromophores, lesdits groupements chimiques chromophores absorbant les rayons ultraviolets lorsque mélangés audit matériau.

4. Inhalateur selon la revendication 3, dans lequel lesdits groupements chimiques chromophores comprennent un ou plusieurs des éléments suivants : Ethene, 1-Hexyne, Ethanal, Nitromethane, bromure de méthyle, iodure de méthyle.

## Patentansprüche

1. Trockenpulverinhalator (100; 200), umfassend einen Körper (110; 210), der eine Verteilungskammer (111; 211) bildet, eine Ausgabeöffnung (131; 231), durch welche hindurch der Benutzer inhaliert, eine Beschickungsöffnung (121; 220), die eine Kapsel (10) aufnimmt, die eine Dosis Trockenpulver zum Inhalieren enthält, und Öffnungsmittel für die Kapsel (140; 265), um eine Kapsel zu öffnen, die in die Beschickungsöffnung eingeführt ist, und die Dosis Pulver in die Verteilungskammer zu leeren, **dadurch gekennzeichnet, dass** der Körper (110; 210) zumindest auf Höhe einer Wand der Verteilungskammer aus einem durchsichtigen gefärbten Material gefertigt ist, das dazu geeignet ist, zumindest teilweise ultraviolette Strahlen zu filtern und es zu ermöglichen, das Innere der Verteilungskammer zu sehen, wobei es zumindest teilweise Rückstände von Pulver verbirgt, die an der mindestens einen Wand aus durchsichtigem gefärbtem Material haften.

2. Inhalator nach Anspruch 1, wobei das gefärbte Material einen Absorptionskoeffizienten aufweist, der im ultravioletten Wellenlängenbereich erhöht ist, typischerweise zwischen 10 und 400 nm.

3. Inhalator nach Anspruch 1 oder 2, wobei dem Material mindestens ein Pigment zugegeben ist, um es zu färben, wobei das mindestens eine Pigment chemische farbgebende Gruppierungen umfasst, wobei die chemischen farbgebenden Gruppierungen ultraviolette Strahlen absorbieren, wenn sie dem Material beigemischt sind.

4. Inhalator nach Anspruch 3, wobei die chemischen farbgebenden Gruppierungen eines oder mehrere der folgenden Elemente umfassen: Ethylen, 1-Hexin, Acetaldehyd, Nitromethan, Methylbromid, Methyliodid.

## Claims

1. A dry-powder inhaler (100; 200) comprising: a body (110; 210) forming a dispersion chamber (111; 211); a dispenser orifice (131; 231) through which the user inhales; a loading opening (121; 220) that receives a capsule (10) containing a dose of dry powder for inhaling; and capsule opening means (140; 265) for opening a capsule inserted into said loading opening and emptying said dose of powder into said dispersion chamber; said inhaler being **characterized in that** said body (110; 210), at least at one wall of said dispersion chamber, is made of a tinted transparent material that is adapted to filter UV rays, at least in part, and to make it possible to see the inside of the dispersion chamber while masking, at least in part, residues of powder that are stuck to said at least one wall of tinted transparent material.

2. An inhaler according to claim 1, wherein said tinted material has an absorption coefficient that is high in the UV wavelength range, typically about 10 nm to 400 nm.

3. An inhaler according to claim 1 or claim 2, wherein at least one pigment is added to said material so as to tint it, said at least one pigment including chromophore chemical groups, said chromophore chemical groups absorbing UV rays when mixed with said material.

4. An inhaler according to claim 3, wherein said chromophore chemical groups comprise one or more of the following elements: ethene; 1-hexyne; ethanal; nitromethane; methyl bromide; methyl iodide.
